# EUROPEAN PATENT APPLICATION

(11) **EP 1 029 511 A1**
(43) Date of publication of application: **23.08.2000**
(21) Application number: 00109148.7
(22) Date of filing: 11.06.1999
(51) Int. Cl.: A61B 18/14

(54) **Medical device for transmyocardial revascularization**

(30) Priority: 10.07.1998 US 113382
(62) Divisional of application: 99928563.8
(71) Applicant: MEDTRONIC, INC., Minneapolis, Minnesota 55432-3576 (US)
(72) Inventor: Darius, Harald, 55271 Stadecken-Ellsheim (DE); Dietz, Ulrich, 65185 Wiesbaden (DE)
(74) Representative: Hughes, Andrea Michelle

(57) **Abstract**

A medical device and method for transmyocardial revascularization. The device features a catheter delivered needle for piercing heart tissue; an RF energy source coupled to the needle; and a controller to control the RF energy source so that a predetermined temperature is reached in the tissue adjacent the device. The controller may also limit the time for which the RF energy is delivered. The needle used is cylindrical and has a piercing length of between approx. 3 to 9 mm and a maximum piercing diameter of between approx. 0.5 to 1.5 mm. In a further embodiment the device features an ultrasonic sensor for sensing the distance from the catheter distal end to a surface of the heart. Additionally the device may feature the ability to deliver a pharmacological agent including physiologic mediators, e.g. growth factors or their respective RNA, DNA or cDNA to the outer surface of the needle. Also disclosed is a method of TMR in a body organ through the use of needle delivered RF energy.

## Description

This invention is generally directed to the field of surgery, and more particularly to surgery procedures to improve the flow of blood to the heart muscle.

The number and variety of medical methods available to repair the effects of cardiovascular disease has increased rapidly over the last several years. More particularly, alternatives to open heart surgery and cardiovascular by-pass surgery have been extensively investigated, resulting in non-surgical procedures such as percutaneous transluminal coronary angioplasty, laser angioplasty, and atherectomy. These procedures are primarily directed toward the reduction of stenosis within the vasculature of a patient by either expanding the lumen through the use of a balloon, or ablating or otherwise removing the material making up the stenosis.

While these procedures have shown considerable promise, many patients still require bypass surgery due to such conditions as the presence of extremely diffuse stenotic lesions, the presence of total occlusions and the presence of stenotic lesions in extremely tortuous vessels. Also, some patients are too sick to successfully undergo bypass surgery, and because the above treatments require surgical backup in the case of complications, they are untreatable. Some patients requiring repeat bypass surgeries are also untreatable. Additionally, patients are untreatable who do not show angiographic evidence of coronary arteries of sufficient internal diameter to serve as target vessels for bypass implantation. Otherwise, only heart transplantation is regarded as therapeutic measure for this type of patients.

One alternative to these procedures is known as Trans-Myocardial Revascularization (TMR). In TMR, channels are formed in the ventricle wall. These channels theoretically will provide blood flow directly from the left ventricular chamber to the ischemic heart muscle. A history and description of this method is presented by Dr. M. Mirhoseini and M. Cayton in "Lasers in Cardiothoracic Surgery" in Lasers in General Surgery (Williams & Wilkins; 1989) pp. 216-223.

To date, the most common clinical experimental approach is forming such channels with a laser. See, for example, U.S. Patents No. 5,755,714 (Murphy-Chutorian) or 5,380,316 (Aita et al.) Such attempts, however, have not been successful. In particular, most channels formed by a laser are thereafter occluded by the body's inflammatory response. This means that the channels are no longer available to deliver the blood to the surrounding tissue.

Besides laser based TMR, others have proposed mechanical or heat or both to form the channels, see for example European Patent Application EP 0 829 239 A1 ((Mueller). Such attempts have also, not to date, been successful. McKenna et al reported, in the Abstract from the 70th Scientific Sessions of the American Heart Association published in Circulation '96, No. 8, Suppl. I, pages 1-217, the use of RF energy to form the channels. They reported, however, that the results were highly similar to that of laser-based TMR, is the channels were occluded. Notably, McKenna et al failed to report the use of temperature control for the delivered RF.

Broadly, it is the object of the present invention to provide an improved method for performing TMR.

It is a yet further object of the present invention to provide a method for performing TMR which results in channel formation which chronically permits increased blood circulation in the region of tissue adjacent the channels.

These and other objects of the present invention will be apparent to those skilled in the art from the following detailed description and the accompanying drawings.

The present invention provides a device for creating channels in a organ comprising
means for piercing heart tissue;
means for delivering RF energy to the means for piercing;
means for controlling the means for delivering RF energy to the means for piercing so that a predetermined temperature is reached in the tissue adjacent the. means for piercing heart tissue.
Additionally the device may feature the ability to deliver a pharmacological agent including physiologic mediators, e.g. growth factors or their respective RNA, DNA or cDNA to the outer surface of the needle. Also disclosed is a method of TMR in a body organ through the use of needle delivered RF energy.

Preferred embodiments will now be described, by way of example only, with reference to the drawings.
FIG. 1 is a view of a first embodiment of the present invention disposed within the human body and particularly accessing the endocardial surface of the left ventricle.
FIG. 2 depicts the distal tip 10 of catheter 1 used in the present invention.
FIG. 3 depicts an alternative embodiment of the present invention.
FIG. 4 shows the end view of the design shown in FIG. 3.
FIG. 5 shows a further embodiment of the present invention.
FIG. 6 depicts the steps used and the method of the present invention.
FIG. 7 depicts a further embodiment of the present invention.
FIG. 8 depicts a further embodiment of the present invention.
FIG. 9 depicts a further embodiment of the present invention.

The FIGS are not necessarily to scale.

### DETAILED DESCRIPTION OF THE INVENTION

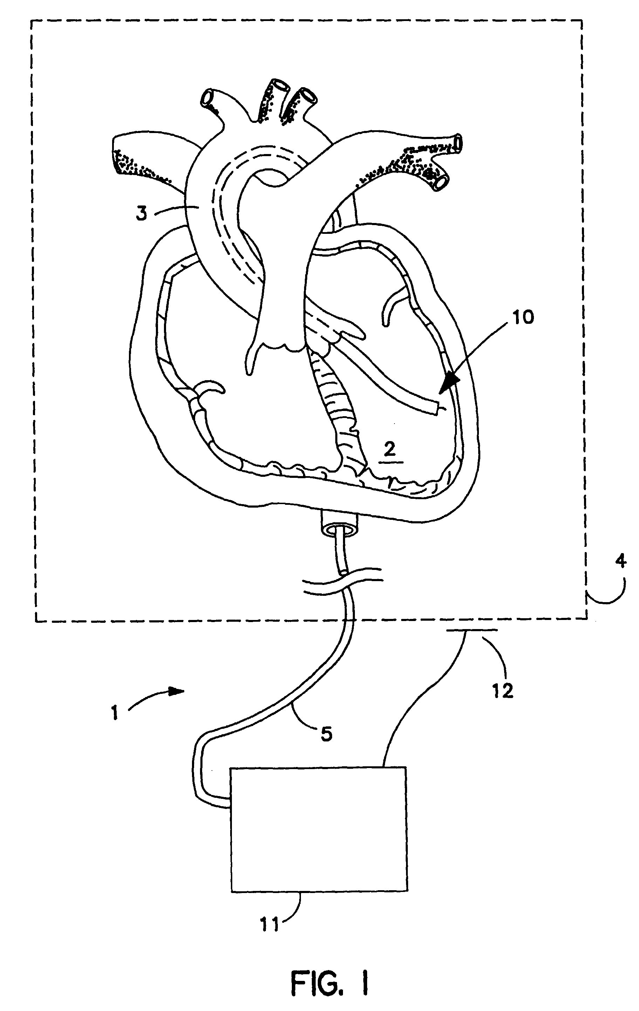

FIG. 1 is a view of a first embodiment of the present invention disposed within the human body and particularly accessing the endocardial surface of the left ventricle. As seen, catheter 1 is introduced into the left ventricle via the aorta 3. This is essentially, in a standard fashion, a femoral percutaneous access through the patient's outer surface 4. Although percutaneous femoral accessing is the preferred method of introducing a catheter of the present invention, other methodologies may also be used such as a direct surgical cut down which exposes the exterior or, in fact, the interior portion of the left ventricle. As seen, catheter 1 features a catheter body 5 and a distal tip 10. Catheter body 5 is of standard design and typically is fashioned of a biocompatible polymeric sheath using one or more conductors and guide lumens therein. The particular design of the catheter body 5 depends on the particular design of the distal tip 10 (discussed in more detail below) as well as the particular handling characteristics the individual physician may desire. For example, the catheter body may be made to be more or less stiff along its entire length or along various portions of its length, as is well known in the art. Moreover, catheter body may be designed so as to be manipulated by either one or more guide lumens contained within the catheter body or one or more guide catheters into which the catheter body may be fitted, or both. Still further, catheter body may be designed in a so-called rapid exchange format permitting the guide wire to be first introduced into the patient's body and the guide catheter manipulated over such wire. The particular design of the catheter body is not within the scope of the present invention and any acceptable design may be used.

As further seen, the proximal end of catheter body couples to a RF energy generator 11 that delivers "Radio Frequency Energy (RF Energy)". In the illustrated embodiment the system operates in a monopolar mode. In this mode the system requires a skin patch electrode 12 that serves as an indifferent second electrode, as is well known in the art. In an alternative embodiment the system could also be operated in a bipolar mode in which the catheter 1 includes two electrodes.

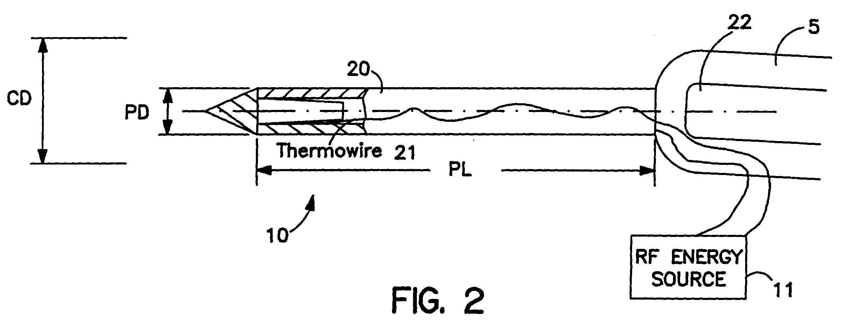

FIG. 2 depicts the distal tip 10 of catheter 1 used in the present invention. As seen, distal tip essentially comprises a needle 20 which is disposed from the distal end of the catheter body 5. Needle is preferably cylindrical and has a sharpened tip. The needle is designed to have a piercing length PL of between approximately 3-9 mm with 7 mm preferred and a maximum piercing diameter PD of between 0.5 - 1.5 mm with 0.7 mm as preferred. As further seen, the needle cooperates with the catheter body, and in particular with the catheter diameter "CD" of the catheter body such that the absolute piercing length of the needle would be no more than the needle length, so long as the catheter body is of sufficient diameter as to be non piercing to the heart under an acceptable force range. In the preferred embodiment the catheter body is at least 3 French in diameter. Of course, the particular dimensions used is highly dependent on both the patient's condition as well as the physician's preference such that any piercing needle or catheter bodies having greater or smaller lengths and diameters may also be used. Moreover, while the needle is shown as made of metal, including the pointed end cap, various portions of the needle need not be metal. For example, the end cap may be glass or polymer. Finally, it is also possible the needle may be provided without any end cap at all.

As further seen, the needle 20 includes within it a temperature measuring device, here illustrated as a thermowire 21. Thermowire permits the temperature of the needle to be reliably measured such that the temperature of the adjacent tissue may be sensed. As seen, thermowire 21 is coupled to RF energy generator 11. Needle 20 is also coupled to RF energy generator 11. Also illustrated in this view is a guide lumen 22 which is disposed within the interior portion of catheter body 5. Guide lumen many be provided to permit a stylet or other control devices to be inserted within catheter and thereby permitting more precise control of needle 20. As already discussed above, however, the particular design of the catheter body may be selected among any of the possible and suitable designs available.

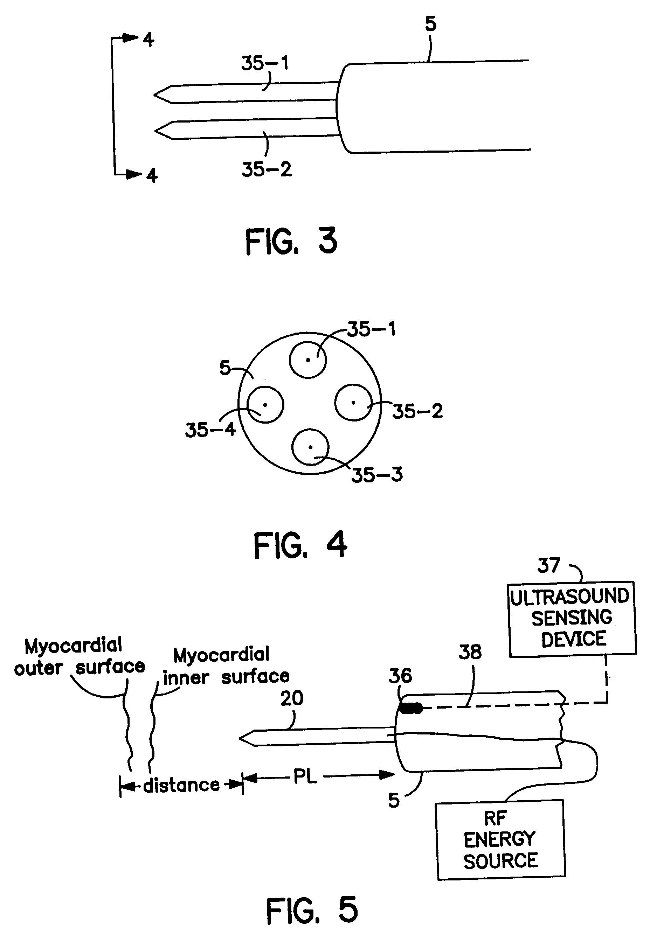

FIG. 3 depicts an alternative embodiment of the present invention. As seen in this embodiment, a multiple number of piercing needles, 35-1 and 35-2 may be disposed away from the distal end of catheter body 5. Each of these piercing needles may further have the dimensions already provided for the design of FIG. 2. Moreover, each of these needles has a similar design, a cylindrical needle having a thermosensing design therein.

FIG. 4 shows the end view of the design shown in FIG. 3. As seen in this view, the embodiment features several needles 35-1, 35-2, 35-3 and 35-4 disposed from the catheter body 5. Through this design a multiple number of channels may be created during a single piercing procedure. While four needles are illustrated, more or less needles could also be used.

FIG. 5 shows a further embodiment of the present invention. As seen in this embodiment, distal end of catheter 5 features a piercing needle 20. Catheter and piercing needle 20 have a design similar to those already discussed in FIG. 2 but for a further addition of an ultrasonic sensor 36 at the distal end of catheter body. As seen, ultrasonic sensor 36 is coupled to ultrasonic sensing device 37 by line 38. Ultrasonic sensor and sensing device may be designed in any acceptable manner including those seen in the Ferek-Petric patents, WO 9517131, WO 9526677, EP 740565, EP 739183, WO 9519201, WO 9515784, EP 474957 each incorporated here by reference. Sensor 36 and sensing device 37 are provided to permit the catheter to sense the distance between the catheter distal end 36 and the surface of the cardiac tissue. Such surfaces include both inner as well as outer myocardial surfaces when the catheter is used in an endocardial manner. Sensors thus permit the needle to be introduced into the myocardium but without piercing through the myocardium and into the pericardial space. As is well known in the art perforation of the myocardium permits bleeding from the inside to the outside. Such bleeding referred to as pericardial effusion often may lead to complications and it is highly desirable that it be avoided. Ultrasonic sensing device 37 would further include a controller which would permit the distance of the sensed heart surface to be correlated with the total length of the piercing needle 20 used such that the distance from the sharpened tip of the piercing needle to the finer outer surface of the local region of the myocardium may be calculated and displayed for the physician.

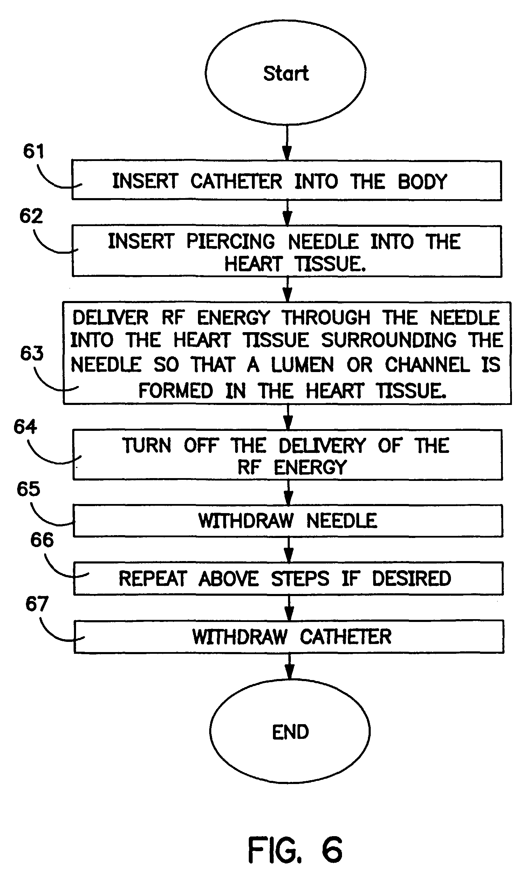

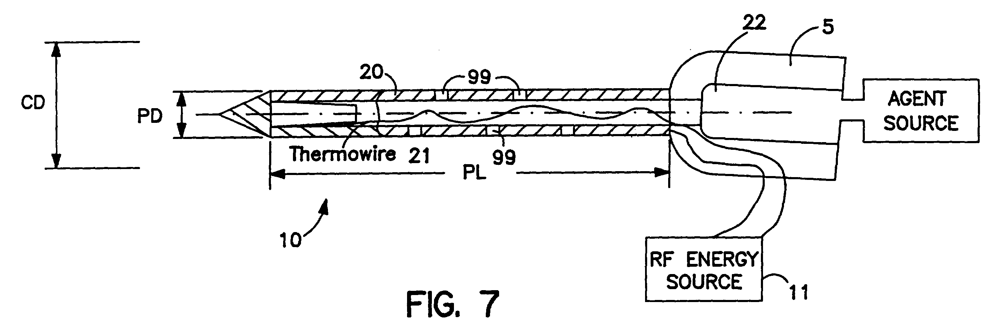

FIG. 6 depicts the steps used and the method of the present invention. At step 61 a catheter having one or more piercing needles as described above is inserted into the body. At step 62 the piercing needle or needles is inserted into the heart tissue. At step 63 RF energy is delivered through the needle into the heart tissue surrounding the needle so that a lumen or channel is formed in the heart tissue. This step would further include the steps of modulating the RF energy delivered in view of the sensed temperature needle so that the temperature of tissue surrounding the needle reaches no more than a predetermined temperature set point. This step would further include the step of only delivering the modulated RF energy for a predetermined period of time between approximately 5 - 25 seconds with 15 seconds preferred. Of course, the particular time at which the energy is delivered depends both upon the patient's physiology, the doctor's preferences as well as the piercing needle's size and geometry and temperature set points used. In the preferred embodiment the temperature set point is between approximately 60°C and 80°C with 70°C preferred. The temperature set point is important as the highest temperature reached by the tissue surrounding the needle determines the amount of necrosis which will form. The inventors believe the temperature ranges given herein will minimize the ultimate zone of necrosis. In step 64 the RF energy is turned off and in step 65 the needle is withdrawn. As seen at step 66, the procedure may be repeated in a further location if desired until finally the catheter is withdrawn at step 67. It should be noted that step 64 may further contain the step of delivering a pharmacological agent through the needle while it is still inserted in the heart tissue. A catheter suitable for such delivery is shown below in FIG. 7. Such pharmacological agents may include vasodilators, anticoagulants, platelet inhibitors, growth factors stimulating angiogenesis or myocyte growth or their respective RNA, cDNA or DNA sequences.

It is also to be understood that step 62 may include a further step of providing a catheter having the steps of sensing the distance of the catheter distal end to one or more surfaces of the heart and calculating and indicating therefrom the distance of the needle distal sharpened tip to the sensed relevant surfaces of the heart so that the physician may reliably control the piercing needle and not cause the myocardium to be entirely pierced by the needle. This step may further include the device emitting a stop forward movement signal to the physician to thereby ensure he is alarmed before transmural myocardial piercing is achieved.

FIG. 7 depicts a further embodiment of the present invention. As seen, this embodiment is substantially the same as that shown in FIG. 2 but for the provision of the agent infusion holes 99 which are in fluid communication with the agent source, as seen. As discussed above in FIG. 6, in a further embodiment the device and method of the present invention may be used to deliver one or more pharmacological agents through the needle while it is still inserted in the heart tissue. Such pharmacological agents may include vasodilators, anticoagulants, platelet inhibitors, growth factors stimulating angiogenesis or myocyte growth or their respective RNA, cDNA or DNA sequences.

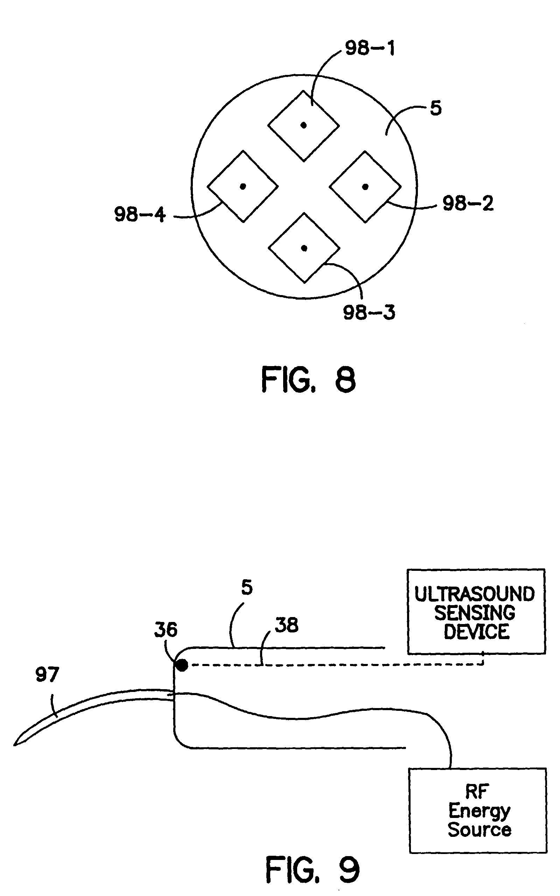

FIG. 8 depicts a further embodiment of the present invention. As seen, this embodiment is substantially the same as that shown in FIG. 4 but for the fact that the needles 98-1 through 98-4 are square.

FIG. 9 depicts a further embodiment of the present invention. As seen, this embodiment is substantially the same as that shown in FIG. 5 but for the fact that the needle 97 is curved along its length and in a single plane. Of course other types of curves may be used, including multi-planar curves, including, for example, a helix.

The entire disclosure of each patent and publication cited herein is incorporated by reference, as if each were individually incorporated by reference.

The invention has been described above in connection with particular embodiments and examples, the invention is not necessarily so limited and that numerous other embodiments, examples, uses, modifications and departures from the embodiments, examples and uses are intended to be encompassed by the claims attached hereto. Variations and modifications can be effected within the scope of the following claims. Such modifications may include substituting elements or components which perform substantially the same function in substantially the same way to achieve substantially the same result for those described herein. For example, although shown in the context of a trans-arterial device, the disclosed invention may also be used in any device configuration, including a hand held direct surgical device as well as remotely operated robotic device.

## Claims

1. A device for creating channels in a organ comprising
means for piercing heart tissue;
means for delivering RF energy to the means for piercing;
means for controlling the means for delivering RF energy to the means for piercing so that a predetermined temperature is reached in the tissue adjacent the. means for piercing heart tissue.

2. The device for creating channels in a organ according to claim 1 further comprising controller means for limiting the controlling means so that the RF energy is delivered for only a predetermined time.

3. The device for creating channels in a organ according to claim 2 wherein the means for piercing heart tissue further comprises a cylindrical needle having a sharpened tip, the needle having a piercing length of between approx. 3 to 9 mm and a maximum piercing diameter of between approx. 0.5 to 1.5 mm.

4. The device for creating channels in a organ according to claim 2 wherein the means for piercing heart tissue further comprises a metal cylinder having a pointed tip.

5. The device for creating channels in a organ according to claim 1 further comprising means for delivering RF energy to the means for piercing comprises

6. The device for creating channels in a organ according to claim 1 further comprising means for delivering a pharmacological agent to the outer surface of the means for piercing whereby the pharmacological agent may be delivered to the surface of the lumen created with the device.
